(19) Europäisches Patentamt
European Patent Office
Office européen des brevets

(11) Publication number: **0 092 286**
**B1**

(12) **EUROPEAN PATENT SPECIFICATION**

(45) Date of publication of patent specification: **08.04.87**

(51) Int. Cl.$^4$: **C 07 D 499/00**

(21) Application number: **83200542.5**

(22) Date of filing: **15.04.83**

(54) **A process for the preparation of penicillanic acid 1,1-dioxide and derivatives thereof.**

(30) Priority: **19.04.82 GB 8211301**

(43) Date of publication of application:
**26.10.83 Bulletin 83/43**

(45) Publication of the grant of the patent:
**08.04.87 Bulletin 87/15**

(84) Designated Contracting States:
**AT BE CH DE FR GB IT LI LU NL SE**

(56) References cited:
**EP-A-0 061 315**
**GB-A-2 045 755**

(73) Proprietor: **Gist - Brocades N.V.**
**Wateringseweg 1 P.O. Box 1**
**NL-2600 MA Delft (NL)**

(72) Inventor: **Henniger, Peter Wolfgang**
**Lammenschansweg 65**
**NL-2313 DK Leiden (NL)**
Inventor: **Van der Drift, Johannes Karel**
**Marterstraat 39**
**NL-2623 ED Delft (NL)**
Inventor: **Kapur, Jagdish Chander**
**Roland Holstlaan 799**
**NL-2524 KB Delft (NL)**
Inventor: **Fasel, Herman Pieter**
**Van der Haertstraat 19**
**NL-2613 XZ Delft (NL)**

(74) Representative: **Van der Straaten, Jan Anthony et al**
**c/o GIST-BROCADES N.V. Patents and Trademarks Department Martinus Nijhofflaan 2**
**P.O. Box 1**
**NL-2600 MA Delft (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the preparation of penicillanic acid 1,1-dioxide and derivatives thereof.

The presumed association between the resistance shown by certain bacteria to betalactam antibiotics and the formation of beta-lactamases has led to an intensive search for beta-lactamase inhibitors.

It is now well-known that penicillanic acid 1,1-dioxide and derivatives thereof having substituents at the beta-lactam methylene carbon atom, and salts and esters thereof, have useful pharmacological properties, for example as effective inhibitors of several types of beta-lactamases present in various kinds of bacteria. They can be administered orally or parenterally.

The present invention is concerned with the preparation of penicillanic acid compounds of the general formula:

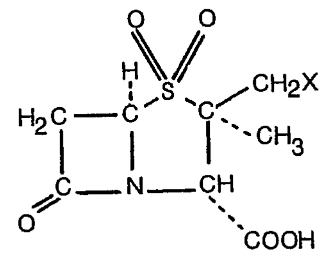

I

wherein X represents a hydrogen or a halogen atom (preferably chlorine, see for example the specification of Dutch Patent Application No. 8100209 and corresponding U.K. Patent Specification 2070592A) or an acetoxy radical and pharmaceutically acceptable salts of such acid or esters of such acids. The compounds of general formula I wherein X represents hydrogen is penicillanic acid 1,1-dioxide (hereinafter referred to for brevity as PAS).

The pharmaceutically acceptable salts referred to above include non-toxic metallic salts such as sodium, potassium, calcium and magnesium, the ammonium salt and substituted ammonium salts, e.g. salts of such non-toxic amines as trialkylamines (e.g. triethylamine), procaine, dibenzylamine, N-benzyl-ß-phenethyl amine, 1-ephenamine, N,N'-dibenzyl-ethylenediamine, dehydroabietylamine, N,N'-bis(dehydro-abietyl)ethylenediamine, N-(lower)alkylpiperidines (e.g. N-ethylpiperidine) and other amines which have been used to form pharmaceutically acceptable salts of penicillins and cephalosporins. The most preferred salts are the alkali metal salts, i.e. the sodium and potassium salts, and the ammonium salts.

The esters of the acids are those which are pharmaceutically acceptable and known in the art to hydrolyze to the free acid *in vivo*. Preferred esters are those with an alkylcarbonyloxymethylene residue in which the hydrogen atom(s) of the methylene unit are optionally replaced by one or two methyl or ethyl groups and the alkyl group contains 1 to 5 carbon atoms, in particular the pivaloyloxymethyl group, or the phthalid-3-yl ester.

The main object of the present invention is to provide an economically viable (possibly "one-pot") industrially applicable process for the preparation of penicillanic acid 1,1-dioxides of general formula I and salts and esters thereof. Such compounds are already known from, for example, the specification of Dutch Patent Application 78 06126 (corresponding U.K. Patent Application 2000138A), United States Patent 4276285, and Dutch Patent Application 8001285 (corresponding U.K. Patent Application 2045755A).

The present invention is concerned with a process for the preparation of penicillanic acid 1,1-dioxides of the general formula:

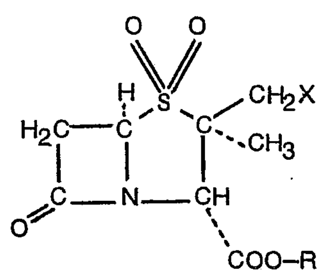

II

wherein X represents a hydrogen or halogen atom or an acetoxy radical and R represents a hydrogen atom or a pharmaceutically acceptable metal or ester radical by debromination of a compound of the general formula:

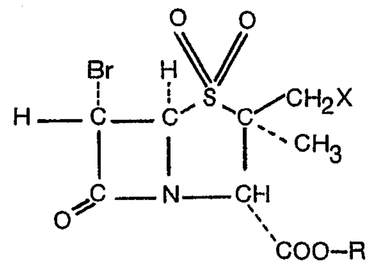

III

and/or of the general formula

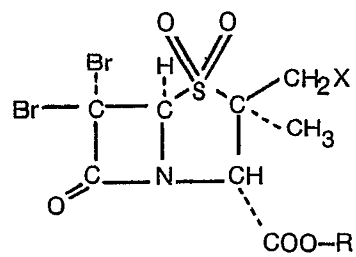

IV

wherein X and R are as hereinbefore defined, by reaction with zinc in association with an acid, and optionally (if R=H) converting the acid into a pharmaceutically acceptable salt or ester, characterized in that the debromination is effected in a water containing medium at a pH of 2.5 to 6 and the acid has a pKa-value measured in water of less than 3.5.

A convenient method to prepare the bromo compounds of formulae III and IV is disclosed in the simultaneously filed patent application entitled "Preparation of 6-alpha-bromo- and/or 6,6-dibromopenicillanic acid 1,1-dioxides", (European application no. 83200541 published under No. 93465).

As mentioned in this simultaneously filed application it is known from Dutch Patent Application 80 01285 (corresponding U.K. Application 2045755A) that 6-alpha-bromo-penicillanic acid is obtained by diazotisation-bromination of 6-beta-amino-penicillanic acid. The Dutch Patent Application 78 06126 (corresponding U.K. Application 2000138A) discloses a reduction (or debromination) step of the bromo-compound with a palladium catalyst and hydrogen to penicillanic acid of the formula:

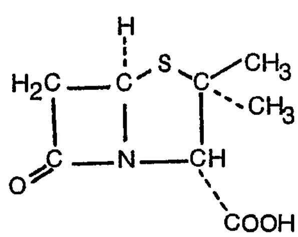

V

and finally the preparation of PAS by oxidation of the penicillanic acid with for example, 3-chloroperbenzoic acid or with a permanganate, e.g. potassium permanganate.

The reduction (or debromination) step indicated in Dutch Application 78 06126 by means of a palladium catalyst and hydrogen is already known from British Patent No. 1072108. As is apparent from this patent as well as from the relevant examples of Dutch Patent Application No. 78 06126, this reduction method is not economic and is highly impractical, as, among other disadvantages, the presence of a thioether linkage, i.e. a sulfide moiety, in the five membered ring of the stuctures of 6-alpha-bromo- and 6,6-dibromo-penicillanic acid necessitates the use of a great amount of catalyst and rather frequent repetition of the reduction sequence with a fresh amount of catalyst, as well as extended reaction times and working under pressures of hydrogen greater than atmospheric. It is therefore not surprising, that even after acquisition of considerable experience in this kind of reaction by the inventors, it was not possible to achieve an average of about 10% overall yield (at best no more than 15%) in many attempts spent on imitation at 0.3 mole scale of the process indicated in Dutch Patent Application 78 06126. (With the term "overall yield" the yield calculated on 6-beta-amino-penicillanic acid as starting material is meant throughout this specification).

In a second series of patent applications, e.g. Dutch Patent Application No. 80 01285 it is disclosed that the overall yield could be increased by reversal of the last two steps. Accordingly, it was found by the inventors that reduction now performed on a mixture of compounds of the formulae:

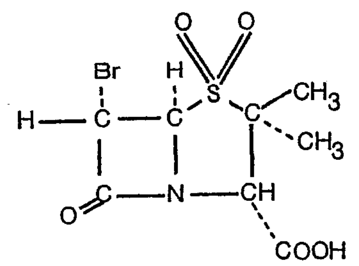

and

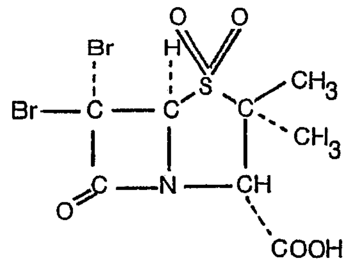

VI VII

in the last step was associated with the use of only about one fifth of the amount of catalyst, working under a pressure above atmospheric but significantly less than that hitherto employed and with less frequent repetition of the reduction sequence. However, although employing a thoroughly worked out procedure for the oxidation with permanganate of 6-alpha-bromo-penicillanic acid to the compounds of formula VI by this method is associated with yields of approximately 80% or better and oxidation of the useful but usually rather small amount of the by-product 6,6-dibromo-penicillanic acid goes with less good yield — the overall yield of PAS (the compound of formula I wherein X represents hydrogen) still could not be raised above a maximum of 20%. It was found as a result of extensive research and experimentation that the poor overall yield is not only caused by the inadequate prior art diazotisation-bromination procedure, but also by the insufficient result of the palladium catalyzed reduction step, and Dutch Patent Application No. 80 01285 (corresponding U.K. 2045755A) mentions other reduction methods such as reduction with a trialkyl tin hydride (e.g. tributyl tin hydride) and also zinc in acetic acid, formic acid or a phosphate buffer.

In view of the necessity to develop an economic and industrially applicable synthesis for PAS, it is apparent that tributyl tin hydride is an expensive reagent, which moreover is less suited for reduction of acid intermediates, while reduction with zinc in a phosphate buffer does not result — so we have found — in better yields than obtained by reduction by means of a palladium catalyst, and reduction with zinc in acetic acid, although producing relatively somewhat tangibly better yields, is not very satisfactory either. As a result of extensive experimentation it has now surprisingly been found that a very easily applicable reduction method for compounds of formulae III and IV which can result in nearly quantitative yields (e.g. 90%) of PAS, is achieved by reduction with zinc and an acid having a pKa-value measured in water of less than 3.5, preferably hydrochloric acid, hydrobromic acid, citric acid or sulphuric acid, at a controlled pH of 2.5 to 6, and preferably 3 to 5. Most preferably hydrochloric acid, hydrobromic acid or sulphuric acid is used.

It will be appreciated by persons skilled in the art that the advantage of this reduction method is clearly shown by the fact that the overall yield calculated on 6-beta-amino-penicillanic acid 1,1-dioxide as starting material from which the bromo-compounds are prepared according to the process of the simultaneously filed application above mentioned is 60—70% (viz the Cignarella diazotisation-bromination procedure discussed in the Dutch application No. 8001285 and an appropriate procedure for the oxidation with permanganate affords and overall yield of 33—38% with a possible maximum of 40%).

The reduction method of this invention will be described in more detail hereafter. One of the major aims in the investigation leading to the present invention was to create a highly rewarding, practical, simple and economic method for the reduction of the bromides of formulae III and IV to the final products of formula II. It will be appreciated that, in view of the prior art disclosure, (for instance in Dutch Patent Applications Nos. 80 01285 and No. 78 06126, and in Dutch Patent Application No. 81 00209 [(corresponding U.K. Application 2070592A) which deals with the preparation of a related compound, i.e. PAS substituted in the beta-methyl group with a chlorine atom by employing in a preferred embodiment a concomitant substitution of the bromine atom and of the 2,2,2-trichloroethyl group (as a protecting group) by hydrogen by means of zinc powder in a mixture of acetic acid and dimethylformamide and giving in a yield of only 37% the desired 2-beta-chloromethyl-2-methyl-penam-3-carboxylic acid 1,1-dioxide starting from 2,2,2-trichloroethyl 6-alpha-bromo-2-beta-chloromethyl-2-methyl-penam-3-carboxylate 1,1-dioxide, as may be derived from e.g. Example XXII, step 4, page 31 in accordance with reaction scheme V of the last formula page,]) any satisfactory reduction method for the replacement of bromine atoms in compounds of formulae III and IV in the present invention will be characterized by narrowly defined conditions. The resulting, unexpectedly and surprisingly effective method of reduction by means of zinc metal, e.g. finely divided zinc powder, is indeed associated with narrowly definable reaction conditions. Thus when R is hydrogen:

—in water, optionally diluted with an inert organic solvent such as acetonitrile, methyl acetate or ethyl acetate, in the latter cases involving reduction in a two-layer system; preferably an amount of at least 5% of water is used;

—at pH 2.5 to 6, preferably between pH 3.5 and 5;

—while adding — preferably in a continuous fashion an acid having a pKa-value in water of less than 3.5, more preferably dilute hydrochloric or hydrobromic acid or sulphuric acid, in order to maintain a pH of 2.5 to 6, preferably 3.5 to 5;

—at a temperature, which may vary between 0 and 20°, but preferably not higher than 15°C for the monobromide and preferably not higher than 10°C for the dibromide of formula IV

—with in general zinc metal, but preferably finely divided zinc powder;

—with, with respect to the number of introduced bromine atoms, about 1.2 to about 2 moles of zinc for the reduction of 1 mole of the monobromide of formula III and about 2.4 to about 4 moles of zinc per mole of dibromide of formula IV, the minimum excess of approximately 20% relating to ideal situations, wherein (nearly) pure compounds of formulae III and/or IV are reduced in relatively concentrated solution, while substantially greater excesses are employed in cases of greater dilution or in cases wherein the number of manipulations after the diazotisation-bromination reaction is reduced considerably.

Acids having a $pK_a$-value in water of less than 3.5 other than dilute hydrochloric, hydrobromic and sulphuric acids which can be employed in conjunction with zinc in the reduction step are, for example, perchloric acid, aryl sulphonic acids (e.g. *p*-tolylsulphonic acid) and sufficiently acidic alkanoic acids, alkanoic diacids (e.g. malonic acid) and citric acid.

Starting from the dibromide of formula IV (R is hydrogen) the conversion yield as well as the actual isolation yield of practically pure compound of formula I is at least 85% and usually about 90%. Starting from the monobromide of formula III (R is hydrogen) the conversion yield is close to 100%, allowing isolation yields of 95% or more of substantially pure product.

If R represents other than hydrogen groups the reaction conditions are essentially the same, except that, although other water-miscible or partly water-miscible solvents such as methyl and ethyl acetate can be employed, the preferred main solvent is acetonitrile containing a sufficient but small amount of water, i.e. about 10% by volume, in order to establish sufficient contact in the heterogeneous reaction between zinc, acid and dissolved brominated substrates. When R represents other groups it is immaterial whether the substrate is a monobromide or a dibromide as in both cases the conversion yields as well as the actual isolation yields of substantially pure product, are well above 85%.

Apart from being highly rewarding, economical and easy to manipulate, this surprisingly efficacious reduction method is also associated with simple isolation procedures as well as with the fortuitous circumstance, that during the reduction by-products are to a large extent converted to virtually non-extractable compounds. Therefore, even when starting from substantially impure bromo-derivatives an uncomplicated extraction procedure already results in substantially purer final products, especially in the case when R is hydrogen.

The aforedescribed particular method for the debromination of 6-bormo- and 6,6-dibromopenicillanic acid derivatives of general formulae III and IV is unique and gives better yields than procedures hitherto disclosed in the prior art, for example that referred to hereinbefore, for the debromination of any 6-bromo- and/or 6,6-dibromopenicillanic acid compounds especially when the reduction reagent is zinc in association with the cheap commonly available hydrochloric acid, hydrobromic acid or sulphuric acid.

The invention is illustrated by the following examples and preparations, however without restricting the scope of this invention.

### Example I

Reduction of a crude mixture of 6,6-dibromopenicillanic acid 1,1-dioxide and 6 alpha-bromo-penicillanic 1,1-dioxide to penicillanic acid 1,1-dioxide.

a. 2,5 g of the crude mixture of bromo-compounds prepared according to example I of the simultaneously filed application were suspended with stirring in an ice-cold mixture of 20 ml of water and 10 ml of acetonitrile. Addition of 4N sodium hydroxide resulted in a clear solution at pH 5.2. Subsequently, 2 g of zinc powder were added with vigorous stirring. At 0—10°C 4N hydrochloric acid was introduced gradually, resulting in a fast reduction of pH to 3.5—4.0. After about 20 minutes the conversion was apparantly completed as was proved by thin-layer chromatography. The excess of zinc was removed by filtration and washed with water. The combined filtrate was somewhat concentrated *in vacuo* in order to remove acetonitrile. The remaining solution in water was extracted repeatedly with ethyl acetate at pH 2. The combined extracts were washed with a small volume of a saturated sodium chloride solution in water, dried over anhydrous magnesium sulphate, filtered, evaporated *in vacuo* and dried extensively *in vacuo.* Yield 1.1 g of a slightly coloured solid.

Analysis of the product by thin-layer chromatography indicated good purity. According to the PMR spectrum of the isolated product, it consisted of penicillanic acid 1,1-dioxide, one unknown byproduct and ethyl acetate in 13:0.7:0.8 molar ratio. This means a purity of at least 90% by weight or about 1.0 g of desired product starting from 2.0 g of starting material and an overall yield of about 55%.

Identification by PMR ($d_6$-DMSO, δ-values in ppm. TMS, 60 Mc): $C(CH_3)_2$: 1.36 (s, 3H) and 1.48 (s, 3H), $C_6$—$H_2$: between 3.08 and 3.9 (octet, $J_{AB}$ 16,2 cps, 2H, $J_{5-6}$ 1.9 cps visible in the high field half and $J_{5-6}$ 4.2 cps visible in the low field half of the splitting pattern), $C_3$—H: 4.27 (s $_1$H) and $C_5$—H: about 5.15 (narrow quartet, $J_{5-6}$ values of about 1.9 and 4.2 cps, 1H).

b. a crude mixture of bromo-compounds (but somewhat further purified than the mixture under a) prepared according to Example 1b of the simultaneously filed application was likewise reduced with zinc, employing conditions somewhat more appropriate to the excess of dibromide present, i.e. reduction at 0—5°C and at pH 4.2 to 4.7. The isolated crude penicillanic acid 1,1-dioxide was more pure than obtained heretofore. The actual overall yield was at least 60%.

Example II

Reduction of a crude mixture of the bromo compounds.

The starting material consisted of the bromo-compounds obtained by diazotisation-bromination of 6-beta-amino-penicillanic acid 1,1-dioxide according to example XI of the simultaneously filed application. After the diazotisation-bromination step the acetonitrile was removed in vacuo, then followed by extractions with dichloromethane and ethylacetate. The combined ethylacetate extract was twice washed with a small volume of saturated sodium chloride solution and subsequently mixed with 150 ml of cold water, whereupon the pH was brought to 3.5 by addition of 4N sodium hydroxyde.

The mixture was vigorously stirred at 10°C and 5 g of zinc powder were added in four portions over 15 minutes, whilst 4N hydrochloric acid was added drop-wise at such a rate that the pH stayed between 3.5 and 4.0. Thereafter 3 g of zinc powder were added and stirring was continued for about 20 minutes until pH correction was no longer necessary. Zinc was removed by filtration through a glass filter reinforced with filter-aid and washed with water and ethyl acetate. The combined filtrate was brought to pH 2.0 with 4N hydrochloric acid, whereupon the layers were separated. The aqueous layer was extracted four times with 150 ml of ethyl acetate, after which the combined extract was washed twice with a small volume of saturated sodium chloride solution, dried over anhydrous magnesium sulphate, filtered, evaporated *in vacuo* and dried extensively at 0.5 mm Hg. Yield 10.0 g of a slightly coloured, largely crystalline product. TLC and the PMR spectrum showed excellent quality with respect to the presence of unknown degradation products.

Example III

Reduction of 6,6-dibromo and 6α-bromo-penicillanic acid 1,1-dioxides with zinc and hydrochloric acid compared with reduction with zinc in a phosphate-citrate buffer

In both experiments were employed the same amount of the same not completely pure mixture of 6,6-dibromo-penicillanic acid 1,1-dioxide and of a much smaller amount of 6α-bromo-penicillanic acid 1,1-dioxide. For as far as possible the reaction conditions and the isolation procedure were kept alike.

(a) A pH 3.6 phosphate/citrate buffer was prepared according to McIlvain starting from 68 ml of an 0.1 molar (21 g per litre) solution of citric acid in water and 32 ml of an 0.2 molar (35.6 g per litre) solution of $Na_2HPO_4.2H_2O$ in water. The actual pH was close to 3.6.

At 6°C a suspension of 3.0 g of the crude mixture of bromides, originating from an homogenized mixture of isolates of several diazotisation-bromination experiments, in 10 ml of ethyl acetate was mixed with vigorous stirring with 100 ml of the above prepared buffer, resulting in a pH of 3.0. From the weighed out amount of zinc powder (6 g) a small portion was added immediately, expecting a quick rise in the pH. Since this did not happen, the pH was raised to 3.5 by addition of a few drops of 4N sodium hydroxide, directly followed by introduction of more zinc. After the introduction of about 3 g of zinc the pH went up to 4.0, continuously operating at 6°C. Next, the remaining amount of zinc was added in one portion. There was no rise of temperature during the reduction. A thin-layer chromatogram prepared 30 minutes after the beginning showed complete conversion of the starting material, but not in a clean fashion. The plate indicated much degradation. The mixture was filtered through glass and the not reacted zinc washed with water. The combined filtrates, having pH 4.0, were brought to pH 2.0 by addition of 4N hydrochloric acid and subsequently extracted four times with 50 ml volumes of ethyl acetate, as checked with TLC resulting in complete removal of penicillanic acid 1,1-dioxide from water. After one washing with 10 ml of a saturated sodium chloride solution, the combined extract was stirred in an ice-bath with anhydrous magnesium sulphate and 0.5 g of activated carbon. After filtration and washings of the filter cake with ethyl acetate, the combined still coloured filtrate was evaporated *in vacuo*, followed by extensive drying *in vacuo*. Yield 0.820 g of coloured product. If the product had been pure, the yield would have been 50%, taking into account the composition and the purity of the starting material.

According to the PMR spectrum of the product, it contained 5 mol% of citric acid and 10 mol% of ethyl acetate. Since other impurities were present too, the actual yield of penicillanic acid 1,1-dioxide was certainly not more than 40%.

(b) To a suspension of 3.0 g of the same starting material in 10 ml of ethyl acetate, cooled in ice, were added with stirring 45 ml of iced water, followed by careful addition of 4N sodium hydroxide until complete dissolution at pH 4.0. Of the weighed out amount of 6 g of zinc powder a small portion was added with vigorous stirring, immediately followed by introduction of 4N hydrochloric acid to pH 3.5. Maintaining the reaction temperature close to 6°C, about half of the amount of zinc was added with 15 minutes while keeping the pH at 4.0 by addition of 4N hydrochloric acid. After 15 minutes reaction the remaining zinc was added in one portion. As under (a) stirring was disrupted after 30 minutes from the start, though the conversion was completed after 20 minutes. TLC showed complete disappearance of the bromides, but also a much more selective formation of penicillanic acid 1,1-dioxide as compared with experiment (a). The spot at the start of the plate, relating to very polar degradation products, was relatively much smaller, and in contrast with the product of experiment (a), an impurity less polar than the desired compound, having a greater Rf value, was now not present. The reaction mixture was treated as described above. The final filtrate was less coloured, so was the crystalline final product. Yield *1.48 g.*, considerably more than obtained above. If the product had been pure, the yield would have been 90.2%. Compared with the product of experiment (a) the appreciably better quality of product (b) allowed for a determination of the

content of penicillanic acid 1,1-dioxide by PMR. This determination gave a purity of 91% by weight. This means a yield of 82.1%, at least twice as much as reached in experiment (a).

By a more diversified extraction procedure it would have been possible of course to obtain a purer product from experiment (a) and a virtually pure product from experiment (b), but such a procedure could have resulted in some losses, eventually leading to a probably still larger difference in the amounts of isolated product. The starting material contained the dibromide and the monobromide in about 9:1 ratio.

Example IV

Further comparison between reduction of 6,6-dibromo and 6α-bromo-penicillanic acid 1,1-dioxides with zinc and hydrohalogenic acids and reduction with zinc in phosphate buffer

The starting material was prepared as follows: A number of isolates obtained from various small scale experiments like those described in Examples VIII and IX of the simultaneously filed application were combined and thereafter suspended in a mixture of ethyl acetate and water. Dilute sodium hydroxide was added until complete dissolution of starting material, thereafter the layers were separated at pH 6.5. The organic layer was discarded and the aqueous layer extracted at pH 2.5 with ethyl acetate. These extracts were combined, washed twice with a small volume of saturated sodium chloride solution, dried over magnesium sulphate, filtered, evaporated *in vacuo* and dried *in vacuo*. This homogeneous and relatively pure mixture of bromides with the dibromide in great excess over the monobromide was analyzed by means of PMR involving addition of a weighed out amount of an internal reference. Of this starting material for the following reduction experiments the exact content and the molar dibromide/monobromide ratio were thereby determined in this manner.

(a) 5 g of the above indicated mixture of bromides were suspended with stirring and cooling in ice in a mixture of 20 ml of ethyl acetate and 40 ml of a phosphate buffer prepared by addition of 10% phosphoric acid to a solution of 35.6 g of $Na_2HPO_4.2H_2O$ in 1 litre of distilled water until pH 6.5. The starting material went into solution resulting in pH 4.0 of the clear mixture. Dilute phosphoric acid was added until the mixture attained pH 3.5. At 4°C 5 g zinc were added over 5 minutes. This did not result in any change in pH or temperature. After addition of 5 g of zinc once more the temperature was raised to 6°C. Reaction was now indicated by a slight drop in the pH and a small rise in temperature. The reaction was disrupted after about 15 minutes additional stirring at 6—8°C. TLC showed complete conversion of starting material; the plate was similar to the one prepared in experiment (a) of Example III, but indicated a slightly more selective reduction. The reaction mixture was treated in the same way as described in Example III. Yield *1.48 g.* of yellowish product, which made a better impression than the material of Example III, experiment (a). From a PMR spectrum taken from a mixture of weighed out amounts of isolated product and a reference compound it could be calculated that the isolated product has a purity of 93.3% by weight. The major impurity was ethyl acetate (3.4% by weight). This means an actual yield of 1.381 g or 47.0%.

The yield, though still rather low, as well as the quality of the product were substantially better than reached in experiment (a) of Example XVIII in the simultaneously filed application referred to above. Since the employed concentration of starting material was not identical in both experiments, it is not justified to conclude that citrate anions are even more deleterious than phosphate anions.

(b) This experiment was an exact as possible repetition of experiment (a), except for one deliberate variation. 1 g of sodium bromide was added to the phosphate buffer in order to establish whether halogen anions could have a catalyzing effect and/or could reduce the obviously deleterious effect of phosphate anions. This was however not apparent from the course of the reduction, nor from TLC, nor from yield and quality of the isolated product. Yield *1.40 g.* Purity determined by means of PMR was only 71.8% by weight. This means an actual yield of only 0.997 g, or 33.9%.

From the experiments of Examples III and IV it is quite clear that phosphate buffers should not be employed in contrast to recommendations proposed in the prior art. From a purely scientific standpoint it could be argued that an increase of ionic strength, such as is apparent in a comparison of experiments (a) and (b) of the present Example, could enlarge the relative share of undesired side reactions, in one way or the other associated with or triggered by a nucleophilic substitution event. In part this may be the case, but then only in part. During experimentation in the development of the present invention it was experienced that reduction applied immediately after preparation of the bromine compounds, thus without intermediate extraction of bromides with ethyl acetate followed by mixing with water, etc., could result in maximal about 20% lower yields i.e., with the same auxiliary agent 45—50% overall yield instead of 55—60%, as well as in the necessity of employing relatively more zinc, as compared with the procedure involving intermediate extraction. However, under such conditions the ionic strength is still greater than applied in the phosphate buffer experiments of Examples III and IV while there may be other origins for the occasionally lower yields of directly applied reductions, such as the presence of substantially more degradation products.

(c) 5 g of the same starting material were suspended with cooling in ice in a mixture of 20 ml of ethyl acetate and 50 ml of cold water. 4N sodium hydroxide were added until complete dissolution at pH 5. About 4N hydrobromic acid was dropped in until the vigorously stirred mixture attained pH 3.8 at 4°C. 4 g of zinc powder were introduced gradually in four portions over 15 minutes, while dilute hydrobromic acid was dropped in to maintain pH 3.5—4. In the beginning the temperature rose to 10°C but settled to 6—8°C later on. After introduction of the last portion of zinc the pH became constant soon, indicating complete

conversion. There was no reason for addition of more zinc, but in order to imitate the conditions of experiments (a) and (b), the mixture was stirred for another 15 minutes at 8—10°C. The usual isolation procedure was followed. Yield 2.46 g. If pure, this weight means a yield of 90.1%. PMR indicated a purity very close to 100%. The actual yield therefore was at least 89%.

(d) This experiment was carried out in somewhat lower concentration as compared with experiment (a) and involved addition of sodium chloride to the phosphate buffer. The ingredients were 3 g of the same mixture of bromides, 20 ml of ethyl acetate, 40 ml of the phosphate buffer, 200 mg of sodium chloride, dilute phosphoric acid for acidification of the mixture to pH 3.7. During the reaction the temperature was directly brought to 6—8°C. 3 g of zinc powder was added in four portions over 15 minutes. Thereafter 2 g of zinc powder were added in one portion, followed by 15 minutes additional stirring at 6—10°C. TLC indicated complete conversion, but also much degradation. Yield *0.820 g.* of semi-solid product, or 46.5% if 100% pure. TLC indicated somewhat less purity as compared with the product of experiment (a). The actual yield was estimated on 40—42%.

## Example V

Comparison of reduction of 6,6-dibromo- and 6α-bromopenicillanic acid 1,1-dioxides with zinc and sulphuric acid and with zinc in phosphoric acid

Since the poor results of reduction with zinc powder in phosphate buffers as shown in the preceding two Examples to some extent could have been caused by the relative high ionic strength in buffers, dilute phosphoric acid is in the present Example compared with sulphuric acid. The starting material was prepared in a larger scale experiment and used without further purification. It contained 84.5% by weight of the dibromide and 6.0% of the monobromide. In 5 g of this product was therefore present 11.77 mmol of useful compound.

(a) To a suspension of 5 g of the crude mixture of bromides in 20 ml of ethyl acetate and 50 ml of iced water 4N NaOH was added dropwise with stirring until a clear solution was reached at pH 5.0. At 8°C 10% phosphoric acid was dropped in until pH 3.8. 4 g of zinc powder were added in four equal portions within 15 minutes at 8—10°C. As in the preceding Examples, it was noticed again that phosphate anions cause an initial lowering of the pH, which was never noticed during reduction in the presence of halogen anions or sulphate anions. By gradual addition of 10% phosphoric acid the pH was kept between 3.5 and 4.0. After the addition of zinc the mixture was stirred additionally during 30 minutes at 8—10°C. After the usual manipulations, 1.88 g of heavily coloured product was obtained. Like the starting material this odorous product was submitted to quantitative analysis by PMR employing weighed amounts of the products and of 3,4,5-trimethoxyphenylacetic acid. If pure the yield would have been 1.88/233/0.01177 times 100% = 68.55%. However the PMR spectrum indicated a purity of maximal 58% as consequence of the presence several degradation products. The actual yield was therefore not better than at best 40%.

(b) Exactly the same experiment was carried out with 4N sulphuric acid. Yield *2.62 g* or 95.54% if pure. Quantitative analysis by PMR indicated a purity by weight of at least 91%. The actual yield was therefore at least 87%.

## Example VI

Preparation of pivaloyloxymethyl penicillanate 1,1-dioxide

A solution of 2,50 g of a mixture of the pivaloyloxymethylesters of 6,6-dibromo- and 6-alpha-bromo-penicillanic acid 1,1-dioxide prepared according to Example XXI of the simultaneously filed application in 50 ml of acetonitrile was cooled to 2°C. 5 ml of cold water and 1.0 g of zinc powder were then added. Subsequently about 10 ml of 1N hydrochloric acid were introduced drop-wise over 5—10 minutes, the rate being adjusted to a pH of not less than 2.5 and a temperature not higher than 8°C. After completion of the addition the pH rose gradually to 6. A thin-layer chromatogram prepared in the meantime showed a clean and complete conversion to the desired compound. The reaction mixture was submitted to filtration through a glass filter strengthened with filter-aid and including washing with acetonitrile. The volume of the combined, almost colourless filtrate was enlarged with 30 ml of cold water, followed by azeotropic removal of acetonitrile *in vacuo*, resulting in the precipitation of an oil. The concentration *in vacuo* was then interrupted in order to introduce a seed crystal. On renewed concentration the oil changed into a crystalline product, which was collected by filtration, washed with cold water and extensively dried *in vacuo* in the presence of phosphorus pentoxide. 1.59 g of according to TLC and the PMR-spectrum, practically pure product were obtained. The yield is at least 88%, as the final product was definitively more pure than the starting material. Yield overall over steps (b) and (c) 48.5%.

IR (KBr-disc, values in $cm^{-1}$): 2990 (m), 1802 (vs), 1778 (vs), 1755 (vs), 1325 (vs), 1280 (m), 1200 (s), 1165 (s), 1110 (vs), 1000 (s), 982 (s).

PMR ($CDCl_3$, 60 Mc, δ-values in ppm, TMS): 1.22 (s, 9H), 1.43 (s, 3H), 1.59 (s, 3H), 3.45 (d, J = 3.3 Hz, 2H), 4.39 (s, 1H), 4.62 (t, J = 3.3 Hz, 1H), 5.65 to 6.00 (AB-q, $J_{AB}$ = 5.4 Hz, 2H).

## Example VII

Comparison between various acids in the reduction with zinc powder

In all experiments there were used the same starting material, that is 3.6 g of a crude mixture of bromides directly obtained from an experiment on larger scale involving the employment of caprolactam

as auxiliary agent. Due to not exhaustive elimination the starting material contained a considerable remaining amount of caprolactam. The final products also contained caprolactam in varying relative amounts. The real content of bromides in the starting material was 3.2 g with a 4:3 molar ratio between the dibromide and the monobromide, or 5.12 mmol of the dibromide and 3.84 mmol of the monobromide. In total 8.96 mmol of useful compounds. The theoretical yield of 100% pure product would then be 2.088 g. All the experiments involved reduction at 8—10°C, a starting mixture of 50 ml of water and 20 ml of ethyl acetate, 2 g of zinc added in 15 minutes in 4 portions and 1 g of zinc added in one portion, followed by 30 minutes additional stirring, except for the last two experiments, wherein stirring was continued during about 2 hours at appreciably higher pH than the pH 3.5 maintained in the first six experiments. Except for the last experiment, in which the acidic mixture was brought to pH 3.5 by addition of solid borax, in all other experiments the pH of the mixture was brought to 5 by addition of 4N NaOH before zinc and acid were introduced. In experiments 2—6 the acid was introduced as an about 10% solution in water. In experiments 7 and 8 the acid was added in solid form because of reduced solubility in water.

| Exp. No. | acid | pH interval | isolated weight | relative content of PAS | actual yield |
|---|---|---|---|---|---|
| 1. | 4N HCl | 3.5—4.0 | 2.24 g | 84.3% | 90.1% |
| 2. | oxalic acid | " | 2.00 g | 79.4% | 76.1% |
| 3. | malonic acid | " | 2.40 g* | 61.6% | 70.8% |
| 4. | citric acid | " | 2.01 g | 90.2% | 86.8% |
| 5. | p-tolyl-sulphonic acid | " | 2.06 g | 73.3% | 72.3% |
| 6. | perchloric acid | " | 2.09 g | 76.7% | 76.8% |
| 7. | 4-chloro-benzoic acid | 4—5.5 | 1.80 g | 57.5% | 49.6% |
| 8. | boric acid | 3.5—6.0 | 1.67 g | 52.7% | 42.2% |

* The product was also contaminated by malonic acid (about 14% by weight)
"PAS" is an abbreviation of penicillanic acid 1,1-dioxide

Overall result:

Phosphoric acid, boric acid and benzoic acids produce poor yields; the latter two also give practical difficulties. Perchloric acid, aryl sulphonic acids and sufficiently acidic alkanoic acids, alkanoic diacids and citric acid can be used in principle, but except for citric acid produce substantially less good yields as compared with hydrochloric acid, hydrobromic acid [cf. Example IV (a)] and sulphuric acid [cf. Example V (b)].

Example VIII

Using the procedure of Example I, 10 g (90% purity—36.3 mmol) of 6-ß-amino-penicillanic acid-1,1-dioxide was converted into a mixture of 6-α-bromo and 6,6-dibromo-penicillanic acid-1,1-dioxides followed by reduction at different pHs during the reduction. The experiment was repeated twice for each pH and the results are reported in the following Table.

| pH | % Yield of PAS |
|---|---|
| 2.0 | 27% (52% unreacted starting material) |
| 3.5—4.0 | 62% |
| 6.5 | 5% (37% unreacted starting material) |

**Claims**

1. A process for the preparation of penicillanic acid 1,1-dioxides of the general formula:

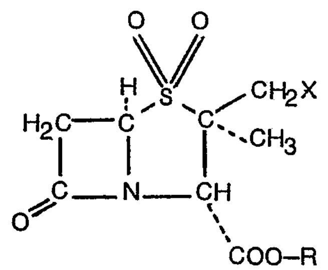

II

wherein X represents a hydrogen or halogen atom or an acetoxy radical and R represents a hydrogen atom or a pharmaceutically acceptable metal or ester radical, by debromination of a compound of the general formula:

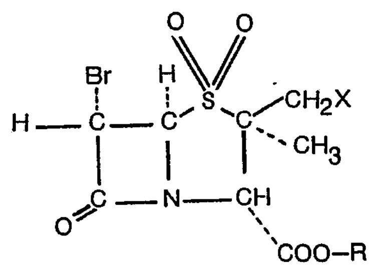

III

and/or of the general formula

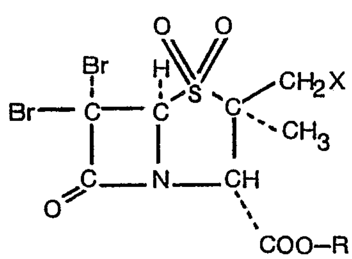

IV

wherein X and R are as hereinbefore defined, by reaction with zinc in association with an acid, and optionally (if R=H) converting the acid into a pharmaceutically acceptable salt or ester, characterized in that the debromination is effected in a water containing medium at a pH of 2.5 to 6 and the acid has a pKa-value measured in water of less than 3.5.

2. The process according to claim 1, characterized in that the water containing medium contains an inert organic solvent.

3. The process according to claim 2, characterized in that the inert organic solvent is acetonitrile, methyl acetate or ethyl acetate.

4. The process according to claims 1—3, characterized in that the pH is 3 to 5.

5. The process according to claim 1 to 4, characterized in that the reduction with zinc is effected in association with diluted hydrochloric acid, hydrobromic acid or sulphuric acid.

6. The process according to claims 1 to 5, characterized in that the reaction is performed at a temperature between 0 and 20°C, preferably between 0 and 10°C.

7. The process according to claims 1 to 6, characterized in that an amount of 1.2 to 2 moles of zinc is used for each bromine atom present in the starting compound.

8. The process according to any one of the preceeding claims in which R = H, characterized in that the penicillanic acid 1,1-dioxide product obtained is converted into its sodium or potassium salt.

**Patentansprüche**

1. Verfahren zur Herstellung von Penicillansäure-1,1-dioxiden der allgemeinen Formel:

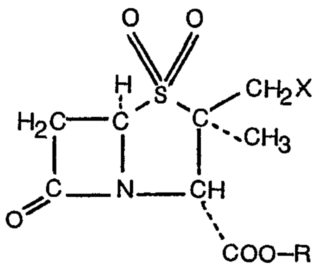

II

worin X ein Wasserstoff- oder ein Halogenatom oder einen Acetoxyrest bedeutet und R ein Wasserstoffatom oder ein pharmazeutisch unbedenkliches Metall oder einen pharmazeutisch unbedenklichen Esterrest bedeutet, durch Entbromieren einer Verbindung der allgemeinen Formel:

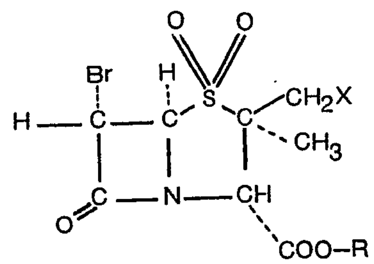

III

und/oder der allgemeinen Formel:

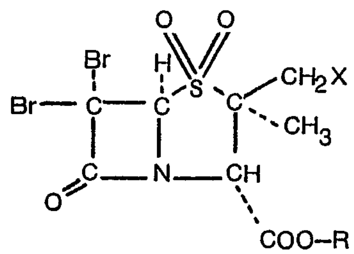

IV

worin X und R wie vorstehend definiert sind, durch Umsetzung mit Zink in Kombination mit einer Säure und gegebenenfalls (falls R = H) Ueberführung der Säure in ein pharmazeutisch unbedenkliches Salz oder einen pharmazeutisch unbedenklichen Ester, dadurch gekennzeichnet, dass das Entbromieren in einem Wasser enthaltenden Medium bei einem pH von 2,5 bis 6 bewirkt wird und die Säure einen pKa-Wert, gemessen in Wasser, von weniger als 3,5 hat.

2. Verfahren nach Anspruch 1, dadurch gekennzeichnet, dass das Wasser enthaltende Medium ein inertes organisches Lösungsmittel enthält.

3. Verfahren nach Anspruch 2, dadurch gekennzeichnet, dass das inerte organische Lösungsmittel Acetonitril, Methylacetat oder Ethylacetat ist.

4. Verfahren nach den Ansprüchen 1 bis 3, dadurch gekennzeichnet, dass der pH-Wert 3 bis 5 beträgt.

5. Verfahren nach den Ansprüchen 1 bis 4, dadurch gekennzeichnet, dass die Reduktion mit Zink in Kombination mit verdünnter Salzsäure, Bromwasserstoffsäure oder Schwefelsäure bewirkt wird.

6. Verfahren nach den Ansprüchen 1 bis 5, dadurch gekennzeichnet, dass die Reaktion bei einer Temperatur zwischen 0 und 20°C, vorzugsweise zwischen 0 und 10°C, ausgeführt wird.

7. Verfahren nach den Ansprüchen 1 bis 6, dadurch gekennzeichnet, dass eine Menge von 1,2 bis 2 Mol Zink für jedes in der Ausgangsverbindung vorhandene Bromatom verwendet wird.

8. Verfahren nach einem der vorangehenden Ansprüche, worin R = H ist, dadurch gekennzeichnet, dass das erhaltene Penicillansäure-1,1-dioxid-Produkt in sein Natrium- oder Kaliumsalz übergeführt wird.

**Revendications**

1. Procédé de préparation de 1,1-dioxydes d'acides pénicillaniques de la formule générale:

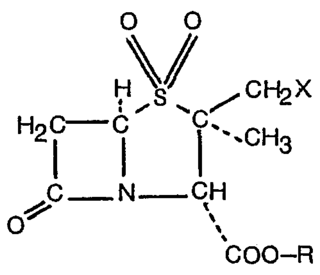

II

où X représente un atome d'hydrogène ou d'halogène ou un radical acétoxy et R représente un atome d'hydrogène ou un métal ou radical ester pharmaceutiquement acceptable, par débromation d'un composé de la formule générale:

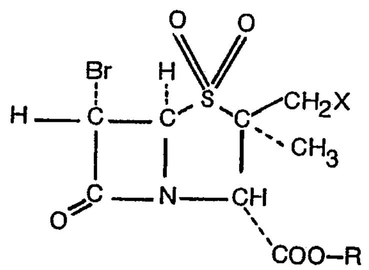

III

et/ou de la formule générale:

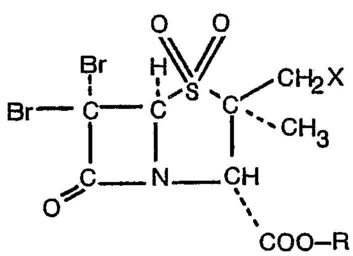

IV

où X et R sont tels que définis ci-dessus, par réaction avec le zinc en association avec un acide, et éventuellement (si R = H) conversion de l'acide en un sel ou ester pharmaceutiquement acceptable, caractérisé en ce que la débromation est exécutée dans un milieu contenant de l'eau a un pH de 2,5 à 6 et l'acide a un pKa, mesuré dans l'eau, de moins de 3,5.

2. Procédé suivant la revendication 1, caractérisé en ce que le milieu contenant de l'eau contient un solvant organique inerte.

3. Procédé suivant la revendication 2, caractérisé en ce que le solvant organique inerte est l'acétonitrile, l'acétate de méthyle ou l'acétate d'éthyle.

4. Procédé suivant les revendications 1 à 3, caractérisé en ce que le pH est de 3 à 5.

5. Procédé suivant les revendications 1 à 4, caractérisé en ce que la réduction avec le zinc est exécutée en association avec de l'acide chlorhydrique, de l'acide bromhydrique ou de l'acide sulfurique dilué.

6. Procédé suivant les revendications 1 à 5, caractérisé en ce que la réaction est exécutée à une température entre 0 et 20°C et de préférence entre 0 et 10°C.

7. Procédé suivant les revendications 1 à 6, caractérisé en ce qu'une quantité de 1,2 à 2 moles de zinc est utilisée par atome de brome en présence dans le composé de départ.

8. Procédé suivant l'une quelconque des revendications précédentes, dans lequel R=H, caractérisé en ce que le 1,1-dioxyde d'acide pénicillanique obtenu est converti en son sel de sodium ou de potassium.